Europäisches Patentamt

European Patent Office

Office européen des brevets

(19)

(11) Publication number: **0 067 000**
**B1**

(12)                          EUROPEAN PATENT SPECIFICATION

(45) Date of publication of patent specification: 23.04.86

(21) Application number: 82302645.5

(22) Date of filing: 24.05.82

(51) Int. Cl.⁴: **C 12 P 19/26,** A 61 K 31/73 //
C12R1/645

(54) Production of a nitrogen-containing polysaccharide having antitumour activity.

(30) Priority: 22.05.81 JP 77789/81

(43) Date of publication of application:
15.12.82 Bulletin 82/50

(45) Publication of the grant of the patent:
23.04.86 Bulletin 86/17

(84) Designated Contracting States:
DE FR GB

(56) References cited:
US-A-4 051 314

CHEMICAL ABSTRACTS, vol. 87, no. 11, 12th
September 1977, page 453, no. 83142t,
Columbus Ohio (USA); J. KNEIFEL et al.:
"Ophiocordin, an antifungal antibiotic of
Cordyceps ophioglossoides"

CHEMICAL & PHARMACEUTICAL BULLETIN,
vol. 25, no. 12, December 1977, pages 3324-
3328, Tokyo (JP); T. MIYAZAKI et al.: "Studies
on fungal polysaccharides. XX.
Galactomannan of Cordyceps sinensis"

(73) Proprietor: SNOW BRAND MILK PRODUCTS
CO. LTD.
36-108 Naebo-cho 6-chome Higashi-ku
Sapporo.shi Hokkaido (JP)

(72) Inventor: Omori, Toshihiro
2-6-75 Chiyoda Sakado-shi
Saitama-ken (JP)
Inventor: Wakizaka, Yoshio
506-5-707 Tokorozawa Tokorozawa-shi
Saitama-ken (JP)
Inventor: Tamura, Kohji
Yukijirushi Dokushin-Ryo 5-11-3 Arajuku-cho
Kawagoe-shi Saitama-ken (JP)
Inventor: Miyata, Nobuo
4-5-8-506 Midori-cho Tokorozawa-shi
Saitama-ken (JP)

(74) Representative: Woods, Geoffrey Corlett et al
J.A. KEMP & CO. 14 South Square Gray's Inn
London WC1R 5EU (GB)

(56) References cited:

CHEMICAL ABSTRACTS, vol. 89, no. 21, 20th
November 1978, page 281, no. 176042s,
Columbus Ohio (USA); S.E. GORIN et al.:
"Extracellular polysaccharides and taxonomy
of Lipomyces yeast cultures"

CHEMICAL ABSTRACTS, vol. 87, no. 21, 21st
November 1977, pages 214-215, no. 163632r,
Columbus Ohio (USA); C.Y.KO et al.: "High-
pressure liquid-chromatographic assay of
nucleotide-pool concentrations during
polysaccharide biosynthesis in four
ascomycetes"

JOURNAL OF THE CHEMICAL SOCIETY, 1951,
pages 2301-2305, London (GB); H.R. BENTLEY
et al.: "Cordycepin, a metabolic product from
cultures of Cordyceps militaris (Linn.) Link. Part
II. The structure of Cordycepin"

CARBOHYDRATE RESEARCH, vol. 101, 1982,
pages 109-116, Elsevier Scientific Publishing
Company, Amsterdam (NL); S. UKAI et al.:
"Structure of a new galactomannan from the
Ascocarps of Cordyceps cicadae SHING"

CHEMICAL ABSTRACTS, vol. 89, no. 2, 10th
July 1978, page 361, no. 12028r, Columbus
Ohio (USA); T. OGATA et al.: "ESR spectra of
various natural fungi with an anti-cancer
effect"

# 0 067 000

**Description**

The present invention concerns a nitrogen-containing polysaccharide having an anti-tumour activity, its production and pharmaceutical compositions containing it.

In recent years, various methods for producing polysaccharides or protein-containing polysaccharides having anti-tumour activity by culturing a *Basidiomycetes* fungus have been reported. Some methods have been practised industrially, for instance United States Patent No. 4,051,314 (Ohtsuka *et al*).

The present inventors, while researching into the anti-tumour activity shown by the polysaccharides produced by microbes, have found that a nitrogen-containing polysaccharide having a high anti-tumour activity is produced by culturing mycelia of *Cordyceps ophioglossoides* Fr. By the way, a fruit body *per se* of *Cordyceps ophioglossoides* Fr. has been customarily used as a roborantia, so-called "vegetative wasp", belonging to *Ascomycetes*.

Accordingly, the present invention provides a nitrogen-containing polysaccharide possessing anti-tumour activity and having the following properties when substantially pure:

(i) appearance: white powdery substance, carbonised with decomposition by heating and not showing a clear melting point;

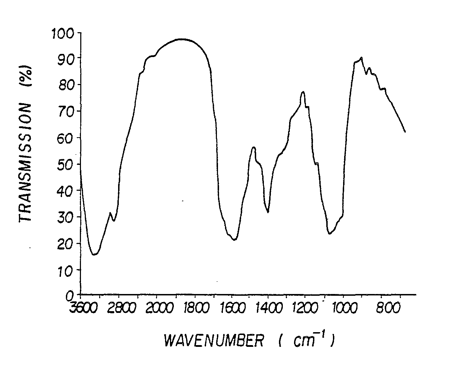

(ii) infrared absorption spectrum: substantially as shown in the accompanying drawing (KBr tablet method);

(iii) solubility: soluble in water and dimethylsulfoxide but insoluble in organic solvents in general;

(iv) colour reaction: positive Molish, anthrone and ninhydrin reactions, and negative Elson-Morgan, Bachat and iodo-starch reactions;

(v) the polysaccharide when in aqueous solution does not permeate a semi-permeable membrane;

(vi) the pH of an aqueous 2 to 10% by weight solution is from 6.2 to 6.4;

(vii) no discernable ultraviolet absorption is obtainable from an aqueous 0.1% by weight solution of the polysaccharide;

(viii) glucose and a minor amount of mannose and galactose are detectable by gas chromatography of the products obtained by hydrolysing the polysaccharide with 2 to 4 N sulfuric acid followed by neutralisation and trimethylsilylisation;

(ix) galactosamine and a minor amount of glucosamine are detectable by amino acid analysis of the products obtained by hydrolysis of the polysaccharide with 4 N hydrochloric acid for 14 hours; and

(x) the polysaccharide comprises polymer chains of neutral sugars and of amino sugars and of aminoacids and contains 40 to 50% by weight of neutral sugars as total hexose by the phenol-sulfuric acid method, 15 to 25% by weight of amino sugars as hexosamine by the indole-hydrochloric acid method and 5 to 15% by weight of protein by the Lowry-Folin method; and

(xi) is obtainable from a culture of *Cordyceps ophioglossoides* Fr.

The invention also provides a process for producing this nitrogen-containing polysaccharide which process comprises culturing at a temperature of 25 to 27°C mycelia of a strain of *Cordyceps ophioglossoides* Fr. in a liquid medium in a stationary state and then in a shaking state or under aeration-agitation, and recovering the polysaccharide thus produced.

Preferably, a strain of *Cordyceps ophioglossoides* Fr. is cultured by agar-plate culture to form mycelia of said fungus, the thus obtained mycelia are cultured in a liquid medium to produce said polysaccharide therein, and said polysaccharide is separated from the liquid medium. Growth of the mycelia in a liquid medium is preferably achieved by culture in a stationary state for 3 to 5 days at a temperature of 25 to 27°C and then in a shaking state or under aeration-agitation for 4 to 6 days at a temperature of 25 to 27°C.

In the description which follows, reference will be made to the accompanying drawing which is an infrared absorption spectrum of the nitrogen-containing polysaccharide obtained by the process according to the present invention.

The strain of *Cordyceps ophioglossoides* Fr. utilized in the present invention is one which produces the desired nitrogen-containing polysaccharide. *Cordyceps ophioglossoides* Fr. is a known species of fungus belonging to genus *Cordyceps* of the family *Hypocreales* of the class Ascomycetes. It is parasitic to *Elaphomyces variegatus* Vittad. and *E. grannulatus* Nees, etc. in soil. It assumes a similar shape to *C. capitata*, however, the head is elliptic to club-like dark-green and later black in colour, and the size is 6—15×4—9 mm. The flesh is yellowish white in colour. The stem is 4—9×0.1—0.3 mm and cylindrical. The base of the stem splits into roots connected to the host body. The perithecium is 0.6—0.7×0.3—0.4 mm. The ascospore splits further into finer and rectangular arthroconidia. A preferred strain has been deposited under deposit number 1FO-8992 at the Institute of Fermentation, Osaka, Japan, which is one of the culture collections recognised for the purposes of the EPC, and under deposit number FERM BP-128 at the Fermentation Research Institute, Agency of Industrial Science and Technology, Japanese Government, which is an International Depositary Authority under the Budapest Treaty.

In a preferred embodiment of the process of the present invention, *Cordyceps ophioglossoides* Fr. 1FO-8992 (FERM BP-128) is at first cultured in an agar plate culture used for culturing a mold fungus or yeast, for instance that prepared by admixing 0.1 to 0.3% by weight of yeast extract with a potato-dextrose agar medium and adjusting the pH to 4 to 7 usually at 20 to 30°C, preferably 25 to 27°C for 10 to 14 days to obtain the proliferated mycelia. Then, the thus obtained white to pale brown mycelia are preferably cultured in a liquid medium as a seed culture generally for 3 to 6 days in a stationary state at the early stage

3

and for 4 to 6 days in a shaking state or under aeration-agitation. The reason why the culture is preferably carried out in this mode is that when the culture is directly carried out in a shaking state or under aeration-agitation, although the proliferation of the mycelium is vigorous, the production of the nitrogen-containing polysaccharide is reduced. The temperature of the liquid culture is 20 to 30°C, preferably 25 to 27°C throughout the two stages.

The medium used in the liquid culture may be any one of the known media used generally for culturing microorganisms, for instance a medium containing glucose as the carbon source to which peptone and yeast extract have been added. In addition, inorganic salts, vitamins or a milk component may be added to the media. The pH of the initial culture medium is suitably 4 to 7. A liquid medium showing a pH of 5.5 before sterilization is particularly preferable for use.

Furthermore, although the culture of the above-mentioned *Ascomycetes* fungus in a liquid medium can be carried out generation after generation, the productivity of the nitrogen-containing polysaccharide by the fungus is reduced with increase in the number of successive cultivations. Accordingly, successive cultivation in liquid culture medium should preferably be limited within only 3 to 5 times. However, from the viewpoint of the productivity of the nitrogen-containing polysaccharide, it is more profitable to utilise a seed culture grown on by stationary culture on an agar plate every time than successively cultivating the liquid culture.

However, accompanying the enlargement of the scale of cultivation, for instance, a liquid culture of more than 10 litres is carried out. It is operationally difficult to use such mycelia obtained by agar-plate culture as the seed culture. Accordingly, the product of the above-mentioned liquid culture in a stationary state may be used as the seed culture.

The mycelia cultured in a liquid medium as mentioned above according to the process of the present invention produce a highly viscous substance and excrete it into the culture medium with the result of making the cultured material highly viscous. In order to recover the nitrogen-containing polysaccharide having an anti-tumour activity from the highly viscous cultured material, a method of extraction is required. However, since direct extraction from the highly viscous cultured material is not effective, it is preferable to carry out the extraction as follows:

First, the cultured material is diluted with 2 to 3 times its weight of distilled water. After heating the diluted material at 80 to 90°C for 30 min., the material is stirred well in a mixer. The thus treated mixture is subjected to centrifugal separation or filtration to obtain an aqueous liquid material from which the nitrogen-containing polysaccharide is collected.

The solid residue of centrifugal separation or filtration is mixed with 2 to 3 times its weight of distilled water and the aqueous mixture is subjected to a crusher, for instance Waring blender to crush the mycelia. After subjecting the aqueous mixture containing the crushed mycelia to centrifugal separation, it is possible to obtain an additional amount of the nitrogen-containing polysaccharide from the thus separated filtrate.

Since the thus obtained aqueous liquid material and filtrate contain various impurities such as free protein, free nucleic acid and reducing sugars, the aqueous liquid and the filtrate are preferably treated with activated carbon or a weakly basic ion-exchanging resin or by chromatography to remove the impurities. By such a treatment, decolorization of the aqueous liquid and the filtrate is effected. In addition, other means of purification of the thus obtained aqueous liquid material and filtrate such as salting out with ammonium sulfate, precipitation with a lower alcohol or acetone, molecular sieving and ultrafiltration may be used additionally either singly or in combination as well as the Sevag method which is used for extracting polysaccharide from cultured materials and the method using enzymes.

After carrying out dialysis of the thus purified aqueous liquid material and filtrate both containing the nitrogen-containing polysaccharide obtained from the cultured material of *Cordyceps ophioglossoides* Fr. against distilled water and concentrating the dialyzate if necessary, the dialyzate or its concentrate is freeze-dried as the nitrogen-containing polysaccharide of the present invention (hereinafter referred to as the present substance).

The steps of purification and those thereafter may be respectively carried out on the aqueous liquid material and the filtrate as mentioned above. However, these steps may be preferably carried out on a mixture of the aqueous liquid material and the filtrate. Further, the aqueous mixture containing crushed mycelia may be directly mixed with the aqueous liquid material without subjecting the aqueous mixture to centrifugal separation, and then the thus obtained mixture may be subjected to the steps of purification and those thereafter.

The thus obtained present substance has the following physical properties:

Appearance: White powdery substance, carbonized with decomposition by heating without showing a clear melting point.

Infrared absorption spectrum: The result of determination using a KBr tablet is shown in the accompanying drawing.

Solubility: Soluble in water and dimethylsulfoxide but insoluble in organic solvents in general.

# 0 067 000

In addition, an aqueous solution of the present substance does not permeate a semipermeable membrane, the pH of an aqueous 2 to 10% by weight solution of the present substance is 6.2 to 6.4 and no recognisable ultraviolet absorption is obtainable from an aqueous 0.1% by weight solution of the present substance.

Colour reaction: Positive Molish, anthrone and ninhydrin reactions, and negative Elson-Morgan, Bachart and iodo-starch reactions.

In addition, glucose and a small amount of mannose and galactose were detected by gas chromatography in the product obtained by hydrolyzing the present substance with 2 to 4 N sulfuric acid followed by neutralization and trimethylsilylization. Further, in the product obtained by hydrolyzing the present substance with 4N hydrochloric acid for 14 hours, galactosamine and a small amount of glucosamine were detected by an aminoacidautoanalyser.

It has been confirmed that the present substance consists of the respective polymers of neutral sugars and amino sugars and aminoacids, as a result of analysis of a supernatant liquid obtained by subjecting to centrifugal separation the well solubilized present substance dispersed in water at a concentration of 1 mg/ml by treatment such as supersonic irradiation. Although the contents of these components depend on the condition of cultivation of the fungus and the conditions of extraction of the cultured material, they are as follows:

Neutral sugars: 40 to 50% by weight as total hexose by the phenol-sulfuric acid method.
Amino sugars: 15 to 25% by weight as hexosamine by the indole-hydrochloric acid method.
Protein: 5 to 15% by weight as protein by the Lowry-Folin method.

Considering of the foregoing characteristics, the present substance obtained according to this invention is believed to be a new nitrogen-containing polysaccharide.

Pharmacological properties of the present substance will be explained as follows:

(1) Acute mammalian toxicity:

As test animals, ICR-JCL mice 4 to 5 weeks old and with a body weight of 20 to 25 g, and Wistar rats 4 to 5 weeks old and with a body weight of 110 to 150 g were used. The present substance was orally or intraperitoneally administered to each animal of groups each consisting of 25 animals at various dose rates. The symptoms, change of body weight and mortality of the animals were observed for a week. Then, all animals were sacrificed and autopsied. The results are shown in the following Table:

TABLE
Acute toxicity of the present substance

| Test animal | Route of administration | $LD_{50}$ mg/kg | |
|---|---|---|---|
| | | Male | Female |
| Mouse | oral | >20,000 | >20,000 |
| | intraperitoneal | >5,000 | >5,000 |
| Rat | oral | >20,000 | >20,000 |
| | intraperitoneal | >5,000 | >5,000 |

(2) Anti-tumour activity:

Examination of the anti-tumour activity of the present substance was carried out on female ICR-JCl mice 6 weeks old and with a body weight of $25\pm3$ g into which had been transplanted cells of Sarcoma-180 solid tumour. Examination of anti-tumour activity was also carried out using 8 week old female mice of the same strain having a body weight of $25\pm3$ g and into which had been transplanted cells of Ehrlich's tumour. The number of animals in each Sarcoma-180 tumour test group was 10 and for each Ehrlich's tumour group was 11.

(a) Anti-tumour activity against Sarcoma-180 tumour:

At the right lower side of each mouse of the test group and the control group was injected subcutaneously 0.1 ml of aqueous physiological saline solution containing about $4\times10^6$ cell of Sarcoma-180 tumour (ascitic type) successively cultured intraperitoneally in a mouse of the same strain as in the test group and after one week of inoculation. Twenty-four hours after transplantation, 0.25 ml of aqueous physiological saline solution containing 5 mg of the present substance per ml of the solution that

5

had been sterilized for 15 min at 120°C was intraperitoneally administered to each mouse of the test group. The same administration was repeated once a day for 10 days running.

0.25 ml of physiological saline solution was administered in the same manner as for the test group to each mouse of the control group.

All mice were sacrificed after 35 days of transplantation to extirpate the solid Sarcoma-180 tumour, which was weighed.

The anti-tumour activity of the present substance against Sarcoma-180 tumour was evaluated by the rate of inhibition on the proliferation of the inoculated tumour cells according to the following formula:

$$\text{Rate of inhibition (\%)} = \frac{C_s - T_s}{C_s} \times 100$$

wherein $C_s$ is the average weight of the tumour in the control group and $T_s$ is the average weight of the tumour in the test group.

As a result of the above-mentioned test, the present substance showed a rate of inhibition of as high as 83.5% and no tumour could be found in five out of the total of 10 animals of the test group.

(b) Anti-tumour activity against Ehrlich's ascites tumour:

$1 \times 10^6$ Cells of Ehrlich's ascites tumour were transplanted into each mouse in 4 groups. Twenty-four hours after transplantation, the present substance was intraperitoneally administered as a solution in aqueous physiological saline at the respective daily dose rates of 25 mg/kg, 10 mg/kg and 2.5 mg/kg to each mouse of the first, second and third groups once a day for 10 days running. Thereafter, the life-prolongation and healing effects of the present substance was evaluated. In addition, only the aqueous physiological saline solution was administered in the same manner as in the other groups to the fourth group (the control group).

The life-prolongation effect was obtained from the following formula:

$$\text{Life-prolongation rate (\%)} = \frac{T_1 - C_1}{C_1} \times 100$$

wherein $T_1$ is the average survival days of the test group and $C_1$ is the average survival days of the control group.

The healing effect was evaluated by the number of cured animals, namely the number of animals which survived for 60 days after the transplantation without retention of ascites.

The results of the test are shown below.

| Group | Dose rate (mg/kg/day) | Life-prolongation rate (%) | Number of cured animal/ total number of a group |
|-------|-----------------------|----------------------------|-------------------------------------------------|
| I | 25 | larger than 200 | 10/11 |
| II | 10 | larger than 200 | 10/11 |
| III | 2.5 | larger than 200 | 9/11 |

As has been shown above, the present substance shows anti-tumour activity against transplanted Sarcoma-180 tumour cells and transplanted Ehrlich's ascites tumour.

When the nitrogen-containing polysaccharide obtained according to the present invention is used as an anti-tumour agent, it can be used by itself or in the form of a pharmaceutical composition also comprising a pharmaceutically acceptable carrier or diluent.

The nitrogen-containing polysaccharide can be administered orally or parenterally as a form of administration unit. It can be administered in the form of, for example, a powder, granules, tablet or capsules for oral administration or injection for parenteral administration.

The following Examples illustrate the present invention.

Example 1

200 ml of a liquid medium of a composition consisting of 10 g of polypeptone, 5 g of yeast extract, 30 g of glucose and one litre of water and adjusted to pH of 5.5 was charged into each of 50 conical flasks of 500 ml capacity. After plugging up the flask with a cotton stopper and sterilizing the flask for 15 min at 120°C, mycelia of *Cordyceps ophioglossoides* Fr. IFO-8992 (FERM BP-128), which had been preliminarily slant-cultured in a potato-dextrose agar culture medium to which has been added a 0.3% yeast extract,

6

# 0 067 000

were inoculated into the culture medium in each flask. The thus inoculated mycelia were cultured in a stationary state for 5 days at 23 to 27°C. They were successively cultured in a shaking state on a rotating table of 180 r.p.m. for 6 days at the same temperature as above to obtain 10 litres of a highly viscous cultured material in total.

Ten litres of hot water were added to the thus obtained cultured material. After stirring the mixture in a mixer, the well-mixed mixture was subjected to centrifugal separation at 10,000 G for 20 min to remove the mycelia in the mixture, thereby obtaining a transparent viscous liquid. A mixture (1:1 by weight) of activated carbon and celite which had been preliminarily washed with concentrated hydrochloric acid, washed well with water, washed with ethanol and then with acetone and dried, was added to the viscous liquid in an amount of 4% by weight. The mixture was stirred for 16 hours at room temperature.

After dialyzing the filtrate of the thus stirred mixture against distilled water for 48 hours at 50°C, the dialyzate was freeze-dried to obtain 25.0 g of a greyish-white powdery substance.

The thus obtained powdery substance was a nitrogen-containing polysaccharide. The anti-tumour activity of the thus obtained substance tested against Sarcoma-180 solid tumour cells transplanted into female ICR-JCL mice 6 weeks old at a daily dose rate of 500 mg/kg body weight for 10 days running showed a rate of inhibition of proliferation of the transplanted tumour cells of as high as 83.5%.

Example 2

100 ml of a liquid medium of a composition consisting of 5 g of polypeptone, 3 g of yeast extract, 30 g of sucrose, each 0.5 g of potassium dihydrogen phosphate and dipotassium hydrogen phosphate, 0.3 g of magnesium chloride, 0.05 g of manganese chloride and one litre of water adjusted to pH of 5.5 was introduced into each of 50 conical flasks of 500 ml in capacity. After plugging the flask with a cotton stopper and sterilizing for 15 min at 120°C, mycelia of *Cordyceps ophioglossoides* Fr. IFO-8992 (FERM BP-128), which had been preliminarily slant-cultured in an agar culture medium, were inoculated into the culture medium in the flasks. The mycelia were cultured in a stationary state for 6 days at 25°C.

On the other hand, 10 litres of a medium of the same composition as above were introduced into a jar-fermenter of 20 litres in capacity. After sterilizing for 30 min at 120°C and cooling, the above mentioned material cultured in the conical flasks was transferred into the culture medium in the jar-fermenter. After culturing the mixture in a stationary state for 2 days at 25°C, it was further cultured under agitation of 250 r.p.m. while blowing air into the medium at a flow rate of 0.4 volumes of the total liquid volume per min for 7 days.

After diluting the thus obtained cultured material with the same volume of distilled water and heating the diluted material at 80°C for 30 min, the mixture was filtered using a filter cloth to remove the mycelia in the cultured material. The thus obtained transparent and highly viscous filtrate was mixed with 10% by volume of Amberlite XAD-2, under agitation for 2 hours at room temperature.

After removing the Amberlite XAD-2 from the mixture, the supernatant liquid was freeze-dried to obtain 25.5 g of a greyish-white powdery substance. The thus obtained powdery substance was a nitrogen-containing polysaccharide mainly consisting of glucose-units. Its anti-tumour activity examined as in Example 1 showed a rate of inhibition of the proliferation of transplanted Sarcoma-180 solid tumour cells of 83.5% at a daily dose rate of intraperitoneal administration of 50 mg/kg.

Example 3

A solution of 0.5 w/v% concentration of the nitrogen-containing polysaccharide (obtained in Example 1) was prepared by dissolving it in 0.01 M of citric acid. The osmotic pressure of the thus obtained solution was adjusted to 1.0 by addition of sodium chloride. The thus obtained solution was sterilized for 15 minutes at a temperature of 120°C to form an injectible pharmaceutical composition.

Administration of the injectible composition against Ehrlich's ascites tumour in mice showed the anti-tumour activity set forth below:

| Dose rate (ml/kg/day) | Life-prolongation rate (%) | Number of cured animal/ total number of a group |
|---|---|---|
| 0.25 intravenously | 32 | 2/11 |
| 0.25 intraperitoneally | >200 | 10/11 |

**Claims**

1. A nitrogen-containing polysaccharide possessing anti-tumor activity and having the following properties when substantially pure:

(i) appearance: white powdery substance, carbonized with decomposition by heating and not showing a clear melting point;

# 0 067 000

(ii) infrared absorption spectrum: substantially as shown in the accompanying drawing (KBr tablet method);

(iii) solubility: soluble in water and dimethylsulfoxide but insoluble in organic solvents in general;

(iv) colour reaction: positive Molish, anthrone and ninhydrin reactions, and negative Elson-Morgan, Bachat and iodo-starch reactions;

(v) the polysaccharide when in aqueous solution does not permeate a semi-permeable membrane;

(vi) the pH of an aqueous 2 to 10% by weight solution is from 6.2 to 6.4;

(vii) no discernable ultraviolet absorption is obtainable from an aqueous 0.1% by weight solution of the polysaccharide;

(viii) glucose and a minor amount of mannose and galactose are detectable by gas chromatography of the products obtained by hydrolysing the polysaccharide with 2 to 4N sulfuric acid followed by neutralisation and trimethylsilylisation;

(ix) galactosamine and a minor amount of glucosamine are detectable by amino acid analysis of the products obtained by hydrolysis of the polysaccharide with 4 N hydrochloric acid for 14 hours; and

(x) the polysaccharide comprises polymer chains of neutral sugars and of amino sugars and of aminoacids and contains 40 to 50% by weight of neutral sugars as total hexose by the phenol-sulfuric acid method, 15 to 25% by weight of amino sugars as hexosamine by the indole-hydrochloric acid method and 5 to 15% by weight of protein by the Lowry-Folin method; and

(xi) is obtainable from a culture of *Cordyceps ophioglossoides* Fr.

2. A process for producing a nitrogen-containing polysaccharide as defined in Claim 1 which process comprises culturing at a temperature of 25 to 27°C mycelia of a strain of *Cordyceps ophioglossoides* Fr. in a liquid medium in a stationary state and then in a shaking state or under aeration-agitation, and recovering the polysaccharide thus produced.

3. A process according to Claim 2, wherein a strain of *Cordyceps ophioglossoides* Fr. is cultured by agar-plate culture to form mycelia of said fungus, the thus obtained mycelia are cultured in a liquid medium to produce the polysaccharide therein, and said polysaccharide thus produced is separated from the liquid medium.

4. A process according to Claim 2 or 3, wherein the mycelia of *Cordyceps ophioglossoides* Fr. are cultured in a liquid medium in a stationary state for 3 to 5 days and then in a shaking state or under aeration-agitation for 4 to 6 days.

5. A process according to any one of Claims 2 to 4, wherein the strain of *Cordyceps ophioglossoides* Fr. is *Cordyceps ophioglossoides* Fr. FERM BP-128.

6. A pharmaceutical composition comprising a nitrogen-containing polysaccharide as defined in Claim 1 or which has been obtained by a process as claimed in any one of Claims 2 to 5 as active ingredient, together with a pharmaceutically acceptable carrier or diluent.

**Patentansprüche**

1. Stickstoff enthaltendes Polysaccharid mit Antitumoraktivität und mit den folgenden Eigenschaften, wenn es im wesentlichen rein ist:

(1) Erscheinen: weiße pulverförmige Substanz, karbonisiert mit Zersetzung durch Erwärmen und keinen klaren Schmelzpunkt zeigend;

(2) Infrarotabsorptionsspektrum: im wesentlichen wie in der beigefügten Zeichnung gezeigt (KBr-Tablettenverfahren);

(3) Löslichkeit: löslich in Wasser und Dimethylsulfoxid, aber in organischen Lösungsmitteln im allgemeinen unlöslich;

(4) Farbreaktion:positive Molish-, Anthron- und Ninhydrinreaktionen und negative Elson-Morgan-, Bachat- und Jod-Stärke-Reaktionen;

(5) das Polysaccharid durchdringt, wenn es in wässriger Lösung vorliegt, eine semipermeable Membran nicht;

(6) der pH-Wert einer wässrigen 2 bis 10 Gew.-%igen Lösung beträgt von 6,2 bis 6,4;

(7) keine erkennbare Ultraviolettabsorption ist von einer wässrigen 0,1 Gew.-%igen Lösung des Polysaccharids erhältlich;

(8) Glukose und eine geringe Menge an Mannose und Galactose sind durch Gaschromatographie der Produkte anzeigbar, die durch Hydrolyse des Polysaccharids mit 2 bis 4 n Schwefelsäure gefolgt durch Neutralisierung und Trimethylsilylierung erhalten wurden;

(9) Galaktosamin und eine geringe Menge an Glukosamin sind durch Aminosäureanalyse der Produkte anzeigbar, die durch Hydrolyse des Polysaccharids mit 4 n Chlorwasserstoffsäure während 14 Stunden erhalten wurden;

(10) das Polysaccharid umfaßt Polymerketten von neutralen Zuckern und von Aminozuckern und von Aminosäuren und enthält 40 bis 50 Gew.-% an Neutralzuckern als Gesamthexose durch das Phenol-Schwefelsäureverfahren, 15 bis 25 Gew.-% an Aminozuckern als Hexoamin durch das Indol-Chlorwasserstoffsäureverfahren und 5 bis 15 Gew.-% an Protein durch das Lowry-Folin-Verfahren; und

(11) ist aus einer Kultur von Cordyceps ophioglossoides Fr. erhältlich.

8

2. Verfahren zur Herstellung eines Stickstoff enthaltenden Polysaccharids nach Anspruch 1, wobei das Verfahren das Kultivieren bei einer Temperatur von 25 bis 27°C eines Myzels eines Stammes von Cordyceps ophioglossoides Fr. in einem flüssigen Medium in einem stationären Zustand und danach in einem geschüttelten Zustand oder unter Durchlüftungsbewegung und Rückgewinnung des so hergestellten Polysaccharids umfaßt.

3. Verfahren nach Anspruch 2 worin ein Stamm von Cordyceps ophioglossoides Fr. durch Agar-Platten Kultivierung kultiviert wird, um ein Myzel des Pilzes zu bilden, das so erhaltene Myzel in einem flüssigen Medium kultiviert wird, um das Polysaccharid darin zu erzeugen und das so erzeugte Polysaccharid aus dem flüssigen Medium abgetrennt wird.

4. Verfahren nach Anspruch 2 oder 3, worin das Myzel des Cordyceps ophioglossoides Fr. in einem flüssigen Medium in einem stationärem Zustand drei bis fünf Tage lang und dann in einem geschütteltem Zustand oder unter Durchlüftungs-Bewegung vier bis sechs Tage lang kultiviert wird.

5. Verfahren nach einem der Ansprüche 2 bis 4, worin der Stamm von Cordyceps ophioglossoides Fr. Cordyceps ophioglossoides Fr. FERM MB-128 ist.

6. Pharmazeutische Zusammensetzung, die ein Stickstoff enthaltendes Polysaccharid nach Anspruch 1 oder ein Stickstoff enthaltendes Polysaccharid, das durch ein Verfahren nach einem der Ansprüche 2 bis 5 hergstellt wurde, als Wirkstoff umfaßt, zusammen mit einem pharmazeutisch verträglichen Träger oder Verdünnungsmittel.

## Revendications

1. Polysaccharide contenant de l'azote possédant une activité antitumeur et ayant les propriétés suivantes lorsqu'il est pratiquement pur:

(i) aspect: substance pulvérulente blanche, qui se carbonise en se décomposant à chaud et ne présente pas de point de fusion net;

(ii) spectre d'absorption infrarouge: pratiquement tel qu'il est représenté dans le dessin ci-joint (méthode à la pastille de KBr);

(iii) solubilité: soluble dans l'eau et le diméthylsulfoxyde, mais insoluble dans les solvants organiques en général;

(iv) réaction colorée: les réactions Molish, à l'anthrone et à la ninhydrine, sont positives et les réactions Elson-Morgan, Bachat et à l'iodo-amidon sont négatives;

(v) le polysaccharide en solution aqueuse ne traverse pas une membrane semi-perméable;

(vi) le pH d'une solution aqueuse de 2 à 10% en poids est de 6,2 à 6,4;

(vii) on ne peut obtenir aucune absorption ultraviolette reconnaissable à partir d'une solution aqueuse à 0,1% en poids du polysaccharide;

(viii) on peut détecter du glucose et une quantité mineure de mannose et de galactose en chromatographiant en phase gazeuse les produits obtenus par hydrolyse du polysaccharide avec de l'acide sulfurique 2 à 4N suivie d'une neutralisation et d'une triméthylsilylisation;

(ix) on peut détecter de la galactosamine et une quantité mineure de glucosamine en analysant les aminoacides des produits obtenus par hydrolyse du polysaccharide avec de l'acide chlorhydrique 4N pendant 14 heures;

(x) le polysaccharide comprend des chaînes polymères de sucres neutres et de sucres aminés et d'aminoacides et il contient 40 à 50% en poids de sucres neutres en tant qu'hexose totale par la méthode au phénolacide sulfurique, 15 à 25% en poids de sucres aminés en tant qu'hexosamine par la méthode à l'indole-acide chlorhydrique et 5 à 15% en poids de protéine par la méthode Lowry-Folin; et

(xi) on peut l'obtenir à partir d'une culture de Cordyceps ophioglossoides Fr.

2. Procédé de production d'un polysaccharide contenant de l'azote suivant la revendication 1, caractérisé en ce que l'on cultive à une température de 25 à 27°C des mycéliums d'une souche de Cordyceps ophioglossoides Fr. dans un milieu liquide à l'état immobile, puis à l'état agité ou sous agitation avec aération, et que l'on récupère le polysaccharide ainsi produit.

3. Procédé suivant la revendication 2, caractérisé en ce que l'on cultive une souche de Cordyceps ophioglossoides Fr. sur une plaque gélosée pour former du mycélium de ce champignon, on cultive les mycéliums ainsi obtenus dans un milieu liquide pour que le polysaccharide se produise dans celui-ci et l'on sépare le polysaccharide ainsi produit du milieu liquide.

4. Procédé suivant la revendication 2 ou 3, caractérisé en ce que l'on cultive les mycéliums de Cordyceps ophioglossoides Fr. dans un milieu liquide à l'état immobile pendant 3 à 5 jours, puis à l'état agité ou sous agitation avec aération pendant 4 à 6 jours.

5. Procédé suivant l'une quelconque des revendications 2 à 4, caractérisé en ce que la souche de Cordyceps ophioglossoides Fr. est le FERM BP-128.

6. Composition pharmaceutique comprenant un polysaccharide contenant de l'azote tel que défini dans la revendication 1 ou obtenu par un procédé suivant l'une quelconque des revendications 2 à 5 à titre de composant actif, avec un support ou un diluant acceptable du point de vue pharmaceutique.